# EUROPEAN PATENT SPECIFICATION

(11) **EP 0 627 209 B1**
(45) Date of publication and mention of the grant of the patent: **07.07.1999**
(21) Application number: 93118205.9
(22) Date of filing: 10.11.1993
(51) Int. Cl.: A61F 13/15, A41B 9/04

(54) **Menses absorbent underpants**
Menstruation aufsaugende Unterhosen
Slip hygiénique absorbant

(30) Priority: 15.11.1992 IL 103753; 30.08.1993 IL 103753
(43) Date of publication of application: 07.12.1994
(73) Proprietor: Vardimon Sless, Hadara, Even Yehuda 40500 (IL)
(72) Inventor: Vardimon Sless, Hadara, Even Yehuda 40500 (IL)
(74) Representative: Kosmin, Gerald Emmanuel

(56) References cited:
- EP-A- 0 249 405
- DE-A- 2 001 659
- GB-A- 1 198 902
- GB-A- 1 356 465
- US-A- 4 743 239

## Description

### FIELD OF THE INVENTION

The present invention relates to menses excretion absorbent means.

### BACKGROUND OF THE INVENTION

All means existing today to absorb menses excretion suffer disadvantages.

Therefore there is an importance and great use for women in a new and ideal invention to absorb menses excretion that is free of the disadvantages.

The existing means and their disadvantages are:
A. Tampons: Cause a feeling of inconvenience, dryness and irritation in the vagina. There are some which are dangerous, that during heavy absorption, their fibers penetrate the blood system through cuts in the vagina, and cause poisoning and death.
B. Cotton wool, or hygienic pads: Cause a feeling of inconvenience, lack of freedom, dryness irritation and rubefaction in the pudenda and between the legs. It is necessary to attach them to the pudenda lips which will hold them. When a woman is moving, they move out of place, and there is a possibility of staining the clothes.
C. Hygienic pads with adhesive strips to the underpants: It is necessary to peel the protective cover off the moist adhesive, to direct and stick the pad right to its place in the underpants. This act is inconvenient and a nuisance. In order to make it work, it is preferred to remove and stretch the underpants. This is hard to perform in public toilets, and especially when the underpants are moist from sweat. The adhesion does not hold together in all situations and the pad moves (for instance while walking or dancing and heavy perspiration). With a pad with a better adhesion, it often happens that only part of the adhesion deteriorates, and then the pad folds in the pudenda and the menses flow to the underpants. And if the pad also turns over when it folds, the pudenda hair sticks and entangles with the moist adhesive that's in it. It also happens that the upper part of the pad sticks to the menses, the lower part is attached to the underpants and then the pad deteriorates and falls apart by the friction produced by walking or movement. When lying on the back, the menses flow backwards, passing the rectum and staining the underpants. Even a pad with an adhesive gives a feeling of lack of freedom and clothes staining is possible. During heavy excretion the menses accumulate at the side of the pad attached to the leg openings in the underpants and the menses flow to the clothing. Even if there was a pad with an excellent adhesion which guarantees no movement, then since the pad is not an integral part of the underpants, it is another layer between the underpants and the pudenda. While walking, dancing, etc., it makes friction and rubbing in the pudenda and between the legs and causes rubefaction. Thus, in any case and even if the pad is thin, there is a feeling of inconvenience and to some extent dryness and irritation in the pudenda and rubefaction between the legs.

These are the existing means designed to absorb menses excretion. As said they have disadvantages and they cause difficulties and bother in addition to natural difficulties which exist during a time of menses excretion.

Another disadvantage of these means is that they offer no solution to a difficult and painful problem of a woman after giving birth, who has stitches in the pudenda, and who, according to doctors' orders, is forbidden to wear regular underpants, for the pudenda area to be ventilated, must hold hygienic pad in the pudenda lips that will support it from falling.

There are also existing disposable diapers designed for babies or old people who cannot control their urine and feces.

These are not designed, and therefore are not suitable, to absorb menses excretion of women. They are not made in the suitable form, or of menses absorbent material. They make the women's figure, which is reflected through the clothing in the pelvis area, appear clumsy and inflated. A woman can't walk about with them and appear in public. It is not possible to disguise their wearing, they are not comfortable for self wearing and for walking. They can not be partially removed like underpants while urinating, etc. Rather, it is necessary to open, remove and to find place for them in the toilets. Also, their closing means are not suitable for opening and reclosing and they get spoiled. There exist furthermore disposable sanitary knickers known for example from document GB-A-1 356 465.

The present invention is free of all the disadvantages which were mentioned above.

### SUMMARY OF THE INVENTION

The invention is an underpant which absorb by themselves, collect, and lock within them menses excretion. The additional effects and the advantages of the invention are:

The invention is in the right form suitable for women's usage and formed of material especially adapted to absorb menses excretion. Unlike regular underpants with an attached pad, which can move with the pad to the sides, letting the clothes be stained, the invention is specially formed to absorb the excretion and not move. It prevents the possibility of staining the clothes in any situation or position, and therefore provides security for perfect appearance. Also there is another advantage that it will not be possible to transfer AIDS by staining a seat. The invention does not inflate the woman's figure which is reflected through the clothing like diapers do, therefore it is possible to disguise its wearing. A woman can walk about with it with convenience and freedom, and to appear in public. It is comfortable for self wearing. It can be partially removed in a toilet. Since the invention is held on the body in the loins and thighs area, the pudenda area can be loose and no rubbing is created in it. It does not cause inconvenient feeling, dryness and irritation in the vagina or in the pudenda. For women after giving birth, with stitches in the pudenda, who cannot tolerate the touch, the pressure or rubbing that comes with conventional means, the invention is an ideal solution. Holes for ventilation can also be made in the invention, so that the pudenda stays ventilated as the doctors order, and the painful necessity to hold the hygienic pad by contracting the pudenda is avoided.

The invention also releases the wearer from the irritating necessity to direct and attach hygienic pads right to their proper place in regular underpants, and releases her from the distress when the pad moves from its place, as often happens. The invention is a ready made product, ready for use, without any moist adhesive residues which might stick to the pudenda hair and entangle with it. It releases the woman from the ridiculous need, when she is in pressure to excrete urine or feces, to look for tweezers and to release with it the pudenda hair that got stuck to the moist glue that's in the hygienic pads, after the pad had folded and turned over in the pudenda.

Since the absorbing element is an integral part of the underpants, the user feels as if she is wearing regular underpants. She does not feel the absorbing element as a foreign body, or have the unpleasant feeling that exists with a pad with adhesion, even if the pad is thin and the adhesion is excellent.

For women during natural abortion, who want to keep the abortion products in order to check whether all the embryo materials are out of the womb, the invention enables accumulating all the abortion products in the underpants and so they can be checked. The invention gives a wonderful feeling of release, relief, freedom, flexibility, and comfort in dressing, like on the other days of the month, when there is no need to wear anything under the clothes besides underpants.

From what was said, the superiority of the invention over all the other means for absorbing menses excretion is obvious.

The technology needed for implementation of the invention is known and existing: Today regular underpants are being manufactured. For the hygienic pads and diapers industry, there are manufactured materials with the ability to seal, to block and prevent passage of menses excretion, materials with the ability to absorb and collect within them menses excretion. There are also known materials with the ability to enable permeation and passage of menses excretion through them, which do not create irritation or damage to the skin, and which are soft and pleasant for the skin to touch, and which protect the absorbing material.

Many of the diapers manufacturers also make the pads, and all are concentrating, and competing with one another, with the quality of the adherence and the thinness of the pad. Despite that pads have so many disadvantages as said, and despite that the invention is the solution which realizes the dreams and expectations of women from menses excretion absorbing means, no one has made the invention, because no one had the inventive spark that exists in the invention.

### BRIEF DESCRIPTION OF THE DRAWINGS

Fig. 1: An example for a figure of menses absorbing underpants with an absorption layer.
Fig. 2: An example for a menses absorbing underpants on a woman's body from the back.
Fig. 3: An example for a menses absorbing underpants on a woman's body from the front.

### DESCRIPTION OF THE PREFERRED EMBODIMENTS

The invention is a self absorbing underpants for menses excretion, hereinafter "menses".

Menses absorbing underpants are short pants which are worn under the outer clothes on the body flesh, and they have as an integral part layer or layers with the ability to absorb within them menses. They have an opening for the loins and two openings for the legs. The openings include an elastic element which ensures that menses will not flow out of the underpants, and that the underpants would not fall down.

They can be worn by threading the legs through the loins opening and the legs openings, and pulling them upwards towards the loins. The underpants configuration is such that their wearing does not inflate the women's figure which is reflected through the clothes, as diapers do. They can be partially removed while urinating etc. like regular underpants. The underpants might also have a layer or layers with a sealing ability, as an integral part on the outer side of the underpants, and then the underpants seal within them menses excretion, even if the absorbing layer is thin.

The underpants might also have a layer or layers with the ability to enable permeation and passage of menses, which are soft and pleasant for the skin to touch, as an integral part in the inner side of the underpants, and then the underpants are soft and pleasant to wear.

This layer has also the ability to protect the absorbing layer from deteriorating and from sticking to the pudenda and to the menses.

Each layer of the underpants can be in the whole area of the underpants, or just in part of their area, which includes the part covering the pudenda and its surrounding, as shown in the drawings. It is also possible to arrange the layers and their edges in a graded and sloping shape, so that the woman's figure continuity that is reflected through tight clothes will be kept.

The technologies which can be used to manufacture the invention are known. Parts of them are implemented in the regular underpants, diapers and the hygienic pads industries. It is possible to manufacture the underpants and to attach the layers that are in them in many ways like glueing, subjugation with heat (heat sealing), sewing, etc., according to the materials from which the underpants are made.

Follows is an example for one of the many possibilities to implement the invention:

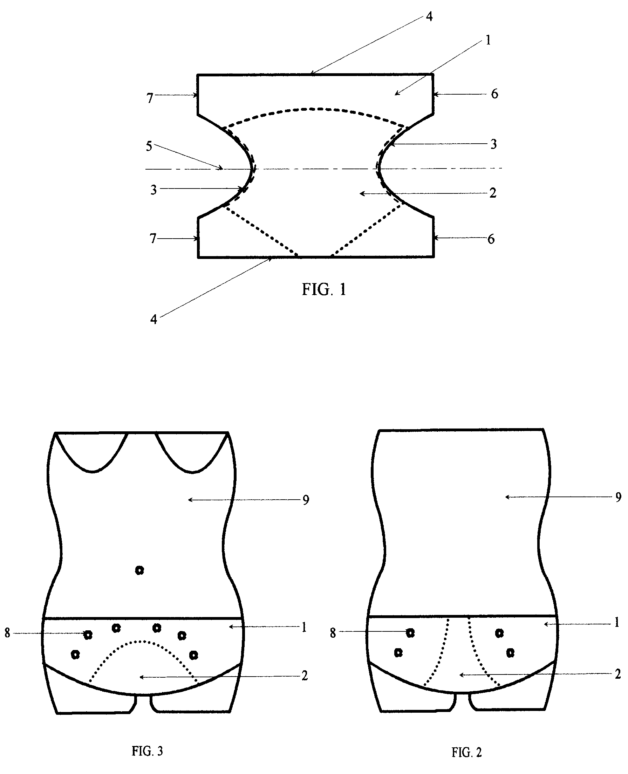

For that we shall be assisted by the enclosed drawings, Figs. 1-3. Take a layer (1) of material with sealing ability in an underpants figure, as Fig. 1. Spray on it glue which is not poisonous and not harmful to human beings. Put on it, on the area that will cover the pudenda, a layer (2) of the material with absorbing ability, in the shape shown in Fig. 1 and having the desired thickness (which is determined by the material's ability to absorb and the desired absorbing capacity). Spray lines of glue as mentioned above on the absorbing layer in a way that doesn't harm much its absorbability. Put on it a layer of the material with the permeation ability and pleasant to touch in the shape (1) of the underpants. Join the layers with subjugation (heat sealing). It is possible to add heat and vacuum to assist with drying the glue and the attachment.

Fold the underpants figure around the folding line (5) in Fig. 1 so that the layer of sealing material points outwards. Sew or glue the corresponding edges (6) with (6) and (7) with (7). Sew or glue a stretched strip of elastic material at the edges of the openings intended for the loins (4) and for the legs (3) so that the desired shrinking will be obtained to provide a smooth, tight fit over the body. It is possible to make holes (8) for ventilation in the underpants in those areas where an absorption layer is not needed, as in Fig. 2 and Fig. 3. The resulting underpants are worn on the woman's body (9) during the menses excretion period, as seen in Fig. 2, from the rear, and Fig. 3, from the front.

It is clear that menses absorbing underpants can be made in various sizes, and figure models, as desired. Similarly the size, shape, and thickness of the absorbing layer can be varied, as desired, so as to fit the underpants for different figures, and in a way that will keep the continuity of the women's figure who wears them. It is also clear that the absorbing capacity of the underpants depends on the capability of the absorbing material to absorb, and on its size. It is clear that when the underpants are made without the sealing layer, they are more airy, suitable for the summer, and for a woman after giving birth. In this case, it is advisable that the absorbing layer have a higher absorbing capacity. It is also clear that when the underpants are made from an absorbing material which is pleasant to the skin to touch, and which does not deteriorate and stick to the menses, it is possible to give up the inner permeable layer mentioned above.

It is also possible to insert in the underpants elements that will enable colosing and opening of the underpants between the legs openings and the loins opening, and thus, it will be possible to remove and put on the underpants also by opening or closing these elements.

## Claims

1. Underpants for use by women during a menstrual period, comprising an undergarment (1) having openings for a wearer's loins and legs respectively, **characterised in that** as an integral part of the undergarment, there is provided:
an outer layer made of a smooth menses sealing material (1), which is pleasant to the touch, in the shape of underpants tailored to conform to one of a plurality of selected body sizes and figure models,
an inner layer made of a smooth menses permeable material which does not stick to menses and which is pleasant to the touch, in the same shape as said outer layer, and
a menses absorbing element (2) coupled between said outer and inner layers, said menses absorbing element having relatively high capacity for absorbing and retaining menses with relatively minimal expansion in thickness,
said menses absorbing element (2) extending at least within the crotch area and lengthwise from the top of the pudendal area of the wearer along the inside of the leg openings to the upper rear edge (4) of the underpants in a thin, flat, not bulky manner,
wherein the edges of said menses absorbing element are formed in a graded, sloping shape so as to form a continuous connection with no sharp boundary with said outer and inner layers, so that the underpants smoothly fit the body of the wearer and do not inflate the figure reflected through tight clothes;
and wherein each of the loins and legs openings, together with the edges of the layers and the element along each opening, are elasticated, so as to prevent leakage of menses through the opening.

2. Underpants for use by women as claimed in Claims 1, **characterised in** **that** as an integral part of the undergarment (1), there is provided a thin menses absorbing element (2) comprising
one or more layers of materials having high capacity for absorbing and retaining menses with minimal expansion in thickness, so as not to inflate the figure of the wearer as seen through tight clothes,
the menses absorbing element (2) being positioned within the crotch area of the undergarment (1) and extending lengthwise at least from the pudendal area of the wearer to the upper rear edge (4) of the undergarment, and being contoured to smoothly fit the body of the wearer, and
a menses permeable layer, which does not stick to menses, as the inner surface of the undergarment.

3. Underpants as claimed in claim 1 or claim 2, wherein the menses absorbing element extends throughout the whole underpants area and is affixed to each of the elasticated openings.

4. Underpants as claimed in Claim 3, wherein the menses absorbing element includes more than one layer, and the layers are arranged in a graded form, with the maximal thickness of the menses absorbing element in the pudenda area, and gradually reduced towards the margins, so as to maintain a smooth, continuous fit over the body.

5. Underpants as claimed in any of the preceding claims and further comprising elements which enable opening and closing of the underpants between the wearer's loins and legs to facilitate wearing and removal of the underpants, the elements being smoothly integrated in the underpants so as not to be visible through tight clothes.

6. Underpants as claimed in any of the preceding claims and further comprising holes (8) for ventilation.

## Patentansprüche

1. Damenschlüpfer der während der Menstruationsperiode getragen werden kann und aus einem Slip (1) mit Öffnungen für die Lenden und Beine einer Trägerin besteht, dadurch gekennzeichnet, daß der Slip folgende integrale Teile aufweist:
eine äußere Lage, die aus einem glatten mensesabdichtendem Material (1) gefertigt ist, das sich geschmeidig anfühlt, wobei der Schlüpfer durch seinen Schnitt für verschiedene Körpergrößen und formen passend ist,
eine innere Lage, die aus einem glatten mensesdurchlässigem Material gefertigt ist, wobei die Lage nicht an Menses haftet, sich geschmeidig anfühlt und die gleiche Form wie die genannte äußere Lage aufweist und
ein mensesabsorbierendes Element (2), das zwischen der genannten äußeren und inneren Lage angeordnet ist, wobei das genannte mensesabsorbierende Element eine hohe Aufnahmefähigkeit und ein hohes Fassungsvermögen aufweist und sich zudem nur minimal verdickt,
wobei das genannte mensesabsorbierende Element (2) sich mindestens bis in den Bereich der Leistengegend und in Längsrichtung von der oberen Grenze des Pudendalbereichs der Trägerin, an den Beinöffnungen entlang bis zum oberen hinteren Rand (4) des Schlüpfers mit einer dünnen, flachen nicht unförmigen Kontour erstreckt,
wobei die Ränder des genannten mensesabsorbierenden Elements in einer abgestuften, schräg abfallenden Form ausgebildet sind, um eine ununterbrochene Verbindung ohne scharfen Übergang zur genannten äußeren und inneren Lage zu bilden, so daß der Schlüpfer sich geschmeidig der Körperform der Trägerin anpaßt und die bei enganliegender Kleidung sichtbare KöArperform nicht vergrößert;
und wobei jede der Lenden- und Beinöffnungen sowie die Ränder der Lagen und des Elements längs jeder Öffnung elastisch sind, um ein Austreten der Menses durch die Öffnung zu verhindern.

2. Damenschlüpfer gemäß Anspruch 1, dadurch gekennzeichnet, daß ein dünnes mensesabsorbierendes Element (2), das einen integralen Teil des Slips (1) darstellt, folgendes aufweist:
eine oder mehrere Lagen Material mit hoher Aufnahmefähigkeit und hohem Fassungsvermögen das sich nur minimal verdickt, so daß die bei enganliegender Kleidung sichtbare Körperform nicht vergrößert wird,
wobei das mensesabsorbierende Element (2) in der Leistengegend des Slips (1) angeordnet ist und in Längsrichtung mindestens vom Pudendalbereich der Trägerin bis zum oberen hinteren Rand (4) des Slips reicht und so geformt ist, das es eng am Körper der Trägerin anliegt und
eine mensesdurchlässige Lage, die nicht wie die Innenfläche des Slips an Menses haftet.

3. Damenschlüpfer gemäß Anspruch 1 oder 2, wobei das mensesabsorbierende Element durch den gesamten Schlüpferbereich verläuft und an jeder der elastischen Öffnungen befestigt ist.

4. Damenschlüpfer gemäß Anspruch 3, wobei das mensesabsorbierende Element aus mehr als einer Lage besteht und die Lagen eine abgestufte Form aufweisen, mit der dicksten Stelle des mensesabsorbierenden Elements im Pudendalbereich und allmählich reduzierend zu den Rändern hin, um einen glatten, durchgehenden Sitz am Körper zu gewährleisten.

5. Damenschlüpfer gemäß jedem der vorstehenden Ansprüche der weiterhin Elemente aufweist mit denen der Schlüpfer zwischen den Lenden und den Beinen der Trägerin geöffnet und geschlossen werden kann, um das Tragen und Ausziehen des Schlüpfers zu erleichtern, wobei die Elemente eben im Schlüpfer integriert sind, so daß sie bei enganliegender Kleidung nicht sichtbar sind.

6. Damenschlüpfer gemäß jedem der vorstehenden Ansprüche mit weiterhin Löchern (8) zur Belüftung.

## Revendications

1. Slip destiné à être utilisé par les femmes pendant qu'elles ont leurs règles, comprenant un sous-vêtement (1) ayant des ouvertures pour les reins et les jambes respectivement d'une porteuse, caractérisé en ce que, comme partie intégrante du sous-vêtement, il est prévu:
une couche extérieure fabriquée à partir d'une matière lisse étanche aux menstrues (1), qui est agréable au toucher, de la forme d'un slip façonné pour s'adapter à l'une d'une pluralité de tailles de corps et de modèles de silhouettes sélectionnés,
une couche intérieure fabriquée à partir d'une matière lisse perméable aux menstrues qui ne colle pas aux menstrues et qui est agréable au toucher, de la même forme que ladite couche extérieure, et
un élément absorbeur de menstrues (2) accouplé entre lesdites couches extérieure et intérieure, ledit élément absorbeur de menstrues ayant une capacité relativement élevée d'absorption et de rétention des menstrues avec une augmentation relativement minime de l'épaisseur,
ledit élément absorbeur de menstrues (2) s'étendant au moins dans la zone de l'entre-jambes et dans le sens de la longueur, du haut de la zone des parties génitales de la porteuse le long de l'intérieur des ouvertures des jambes jusqu'au bord arrière supérieur (4) du slip d'une manière mince, plate, pas encombrante,
dans quoi les bords dudit élément absorbeur de menstrues sont dotés d'une forme inclinée progressive de façon à former une connexion continue sans limite brusque avec lesdites couches extérieure et intérieure, de façon à ce que le slip soit adapté au corps de la porteuse et ne grossisse pas la silhouette que l'on voit à travers des vêtements ajustés;
et dans quoi chacune des ouvertures des reins et des jambes, ainsi que les bords des couches et l'élément le long de chaque ouverture, sont élastiquées, de façon à empêcher la fuite des menstrues à travers l'ouverture.

2. Slip destiné à être utilisé par les femmes comme revendiqué dans la revendication 1, caractérisé en ce que, comme partie intégrante du sous-vêtement (1), il est prévu un élément mince absorbeur de menstrues (2) comprenant
une ou plusieurs couches de matières ayant une capacité élevée d'absorption et de rétention des menstrues avec une augmentation minime de l'épaisseur, de façon à ne pas grossir la silhouette de la porteuse telle qu'on la voit à travers des vêternents ajustés,
l'élément absorbeur de menstrues (2) étant positionné dans la zone de l'entre-jambes du sous-vêtement (1) et s'étendant dans le sens de la longueur, au moins de la zone des parties génitales de la porteuse jusqu'au bord arrière supérieur (4) du sous-vêtement, et dont les contours sont adaptés exactement au corps de la porteuse, et
une couche perméable aux menstrues, qui ne colle pas aux menstrues, en tant que surface intérieure du sous-vêtement.

3. Slip comme revendiqué dans la revendication 1 ou la revendication 2, dans quoi l'élément absorbeur de menstrues s'étend dans toute la zone du slip et est collé à chacune des ouvertures élastiquées.

4. Slip comme revendiqué dans la revendication 3, dans quoi l'élément absorbeur de menstrues comporte plus d'une couche, et les couches sont arrangées de façon progressive, l'épaisseur maximale de l'élément absorbeur de menstrues étant dans la zone des parties génitales, et progressivement réduite vers les marges, de façon à maintenir un ajustement continu et uniforme sur le corps.

5. Slip comme revendiqué dans l'une quelconque des revendications précédentes et comprenant encore des éléments qui permettent l'ouverture et la fermeture du slip entre les reins et les jambes de la porteuse pour faciliter le port et le retrait du slip, les éléments étant uniformément intégrés dans le slip de façon à ne pas être visibles à travers des vêtements ajustés.

6. Slip comme revendiqué dans l'une quelconque des revendications précédentes et comprenant encore des trous (8) pour l'aération.
